# EUROPÄISCHE PATENTSCHRIFT

(11) **EP 1 064 954 B1**
(45) Veröffentlichungstag und Bekanntmachung des Hinweises auf die Patenterteilung: **09.04.2003**
(21) Anmeldenummer: 00250177.3
(22) Anmeldetag: 08.06.2000
(51) Int. Cl.: A61L 2/07

(54) **Dampfsterilisator**
Steam steriliser
Stérilisateur à vapeur

(30) Priorität: 29.06.1999 DE 19930546
(43) Veröffentlichungstag der Anmeldung: 03.01.2001
(73) Patentinhaber: MELAG oHG Medizintechnik, 10829 Berlin (DE)
(72) Erfinder: Hartrodt, Matthias, 16341 Schwanebeck (DE); Waydbrink, Eberhard v.d., 14641 Paulinenaue (DE); Lenz, Olaf, 12209 Berlin (DE)
(74) Vertreter: Gross, Felix, Dr.

(56) Entgegenhaltungen:
- EP-A- 0 937 465
- DE-A- 4 445 054

## Beschreibung

Die Erfindung betrifft einen Dampfsterilisator nach dem Oberbegriff des Anspruchs 1 und ein Verfahren zum Betrieb eines Dampfsterilisators nach dem Oberbegriff des Anspruchs 17.

Es sind Dampfsterilisatoren bekannt, die nach dem fraktionierten Vakuumverfahren arbeiten. Eine solche Vorrichtung ist z. B. aus der DE 44 45 054 A1 bekannt, bei der ein Dampfsterilisator mit einem Druckkessel und einem Kondensator betrieben wird. Der Kondensator weist ein Kondensatsammelgefäß auf, in dem anfallendes Kondensat gesammelt wird. Zur Entleerung wird ein Ventil geöffnet, wodurch sich das Kondensat durch die Schwerkraft in einen Auffangbehälter entleert.

Dabei ist es nachteilig, daß die Schwerkraft unter Umständen nicht ausreicht, um das Kondensat aus dem Dampfsterilisator vollständig zu entleeren.

Der Erfindung liegt die Aufgabe zugrunde, einen Dampfsterilisator zu schaffen, bei dem eine effiziente Entleerung des Kondensates möglich ist.

Erfindungsgemäß wird das entsprechend den kennzeichnenden Merkmalen des Anspruchs 1 erreicht. Durch die Verwendung eines Mittels zur Entleerung des Kondensats durch Überdruck aus dem Kondensator und / oder mindestens einem Kondensatsammelgefäß wird gegenüber der Schwerkraftentleerung eine bessere Entleerung des Kondensates erreicht bzw. eine Entleerung des Kondensates in einen höher gelegenen Behälter überhaupt erst ermöglicht. Das Kondensat wird mit einer höheren Energie aus dem System getrieben, als dies allein mit Schwerkraft möglich wäre. Auch ist damit eine besonders effiziente Kühlung bzw. Kondensation möglich, so daß das Kondensat direkt in eine Abwasserleitung einleitbar ist.

In einer besonders vorteilhaften Ausgestaltung des erfindungsgemäßen Dampfsterilisators ist der Kondensator und /oder das Kondensatsammelgefäß über eine erste Dampfleitung vom Druckkessel mit Überdruck beaufschlagbar. Durch die Verbindung über die erste Dampfleitung kann mit geringem apparativen Aufwand ein Überdruck im Druckkessel dazu verwendet werden, das Kondensat durch eine Druckentleerung abzuführen. Mit Vorteil kann die Druckbeaufschlagung auch über eine externe Druckquelle (z.B. Druckleitung, Druckflasche) erfolgen. Auch können beide Alternativen der Druckbeaufschlagung gleichzeitig angewendet werden, wodurch eine besondere Flexibilität des erfindungsgemäßen Dampfsterilisators erreicht wird.

In einer vorteilhaften Ausgestaltung des Dampfsterilisators ist das Kondensat durch den Überdruck in einen Wasserbehälter und / oder eine Kondensatablaufleitung förderbar. Durch die Rückführung des Kondensates in den Wasserbehälter ist ein geschlossener Wasserkreislauf möglich, wodurch Wasser eingespart wird. Zur Erhöhung der Flexibilität kann das Kondensat gleichzeitig oder alternativ auch über die Kondensatablaufleitung aus dem System geschleust werden.

In einer weiteren vorteilhaften Ausführungsform des erfindungsgemäßen Dampfsterilisators weist der Wasserbehälter einen Abwasserteil und einen Frischwasserteil auf. Mit Vorteil kann rückgeführtes Kondensat in dem Abwasserteil des Wasserbehälters aufgefangen werden.

Vorteilhafterweise stehen der Abwasserteil und der Frischwasserteil über ein Überlaufwehr miteinander in Verbindung. Durch diese Ausgestaltung wird eine baulich einfache Verbindung zwischen den beiden Teilen geschaffen.

In einer weiteren vorteilhaften Ausgestaltung des erfindungsgemäßen Dampfsterilisators ist über die erste Dampfleitung eine Vakuumpumpe mit dem Druckkessel verbindbar. Mit besonderem Vorteil ist die Vakuumpumpe als Membranpumpe ausgebildet.

Mit Vorteil weist der Kondensator Kühlflächen auf, denen mindestens ein Ventilator und / oder eine Wärmepumpe und /oder Peltierelemente zugeordnet sind. Damit ist eine besonders effiziente Kondensation möglich.

In einer Ausführungsform ist die vom Druckkessel ausgehende zweite Dampfleitung unterhalb des Luftkühlers am Kondensatsammelgefäß angeschlossen und ein Kondensatauslaß ist oberhalb des Luftkühlers am Kondensatsammelgefäß vorgesehen. In dieser Ausführungsform verbleibt ständig eine größere Menge Kondensat im Kondensatsammelgefäß, wodurch die Wärmekapazität des Kondensators erhöht und die Kondensationswirkung verbessert wird.

In einer weiteren Ausführungsform sind die vom Druckkessel ausgehende zweite Dampfleitung und der Kondensatauslaß unterhalb des Luftkühlers am Kondensatsammelgefäß vorgesehen.

Zur Unterstützung der Kondensation ist vorgesehen, daß eine Kondensatleitung nach dem Kondensator oder dem Kondensatsammelgefäß durch den Wasserbehälter verläuft und in diesem Bereich als Kondensatorschlange ausgebildet ist.

Weiterhin ist es vorteilhaft, daß Wasser durch eine Speisepumpe aus dem Wasserbehälter und / oder aus einer Wasserzuleitung zum Druckkessel pumpbar ist. Somit kann die Wasserversorgung flexibel unterschiedlichen Betriebspunkten angepaßt werden. Da demineralisiertes oder destilliertes Wasser verwendet wird, kann der Wasserzulauf z.B. mit einer Wasseraufbereitungsanlage verbunden werden.

Auch ist es vorteilhaft, wenn Wasser aus dem Wasserbehälter und / oder aus einer Wasserzuleitung aufgrund einer Druckdifferenz zum Druckkessel förderbar ist. Damit könnte die Wasserversorgung allein mit einem Ventil bewerkstelligt werden. Die Druckdifferen zu könnte sich aus dem statischen Druck eine höherliegenden Wasserbehälters oder aus einem Druck hinter einer Wasseraufbereitungsanlage ergeben.

In einer weiteren besonders vorteilhaften Ausführungsform wird die Druckdifferenz dadurch erhöht, daß die Wassereinspeisung an einem Betriebspunkt erfolgt, an dem der Druckkessel unter Vakuum steht.

Die Aufgabe wird auch durch ein erfindungsgemäßes Verfahren mit den Merkmalen des Anspruchs 17 gelöst. Dabei wird Dampf in einem Kondensator kondensiert, wobei das anfallende Kondensat aus dem Kondensator oder mindestens einem Kondensatsammeigefäß durch Überdruck entleert wird. Damit ist eine Kondensatentleerung auf besonders einfache Weise möglich.

In einer besonders vorteilhaften Ausgestaltung des Verfahrens wird der Überdruck zur Kondensatentleerung aus dem Druckkessel entnommen, so daß ein besonders effizienter Betrieb möglich ist.

Weiterhin ist es vorteilhaft, daß der Überdruck zur Kondensatentleerung aus einer externen Druckquelle stammt. Auch ist eine Druckentleerung durch Kombination einer Druckentleerung durch Überdruck aus dem Druckkessel und einer externen Druckquelle möglich.

Die Erfindung soll in einem Ausführungsbeispiel anhand von Zeichnungen näher erläutert werden. Es zeigen:
- Fig. 1: den schematischen Aufbau eines Dampfsterilisators gemäß der Erfindung;
- Fig. 2: den schematischen Aufbau des erfindungsgemäßen Dampfsterilisators während der Evakuierung des Druckkessels;
- Fig. 3: den schematischen Aufbau des erfindungsgemäßen Dampfsterilisators während der Druckentleerung des Kondensates aus einem Kondensator mit Kondensatsammelgefäß.

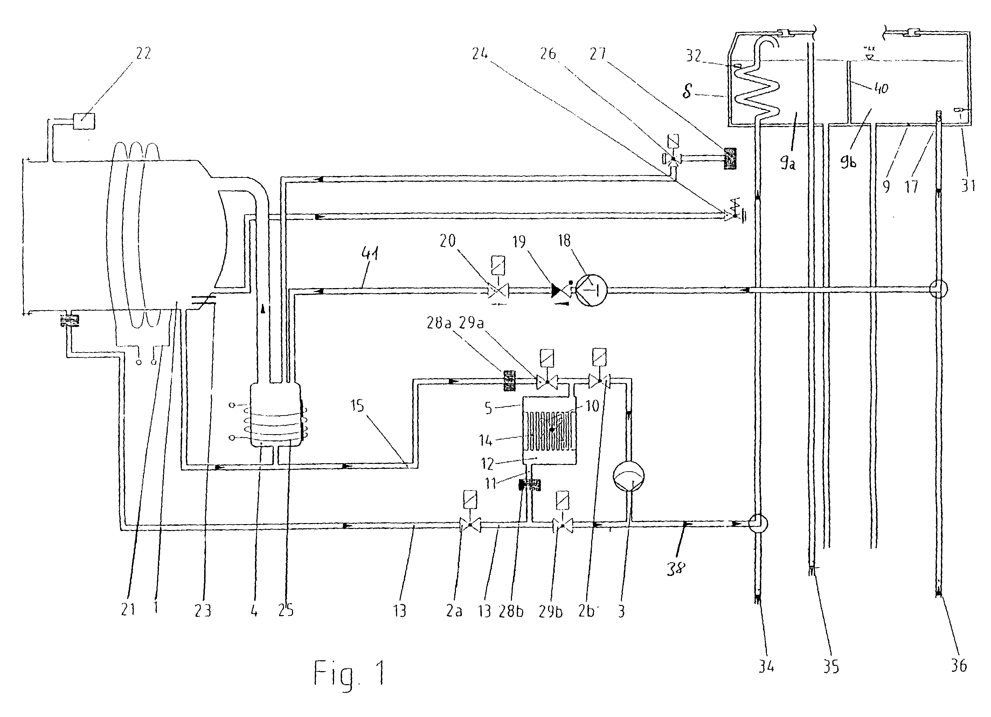

Der in Fig. 1 dargestellte Dampfsterilisator weist einen Druckkessel 1 auf, in dem sich das zu sterilisierende Gut befindet. Dem Druckkessel 1 ist eine Vorheizung 21 zugeordnet, um den Druckkessel 1 zur Vermeidung der Kondensatbildung an der kalten Druckkesselwand vorwärmen zu können. Weiterhin ist der Druckkessel 1 mit einem Drucksensor 22, Temperaturfühlern 23 und einem Federsicherheitsventil 24 ausgerüstet. Dem Druckkessel 1 sind auch eine Membranpumpe als Vakuumpumpe 3 sowie ein Dampferzeuger 4 zugeordnet.

Dem Dampferzeuger 4 sind eine Heizung 25 sowie ein Belüftungs-Magnetventil 26 mit einem Belüftungsfilter 27 zugeordnet. Der Dampferzeuger 4 und der Druckkessel 1 sind mit der ersten Dampfleitung 15 verbunden.

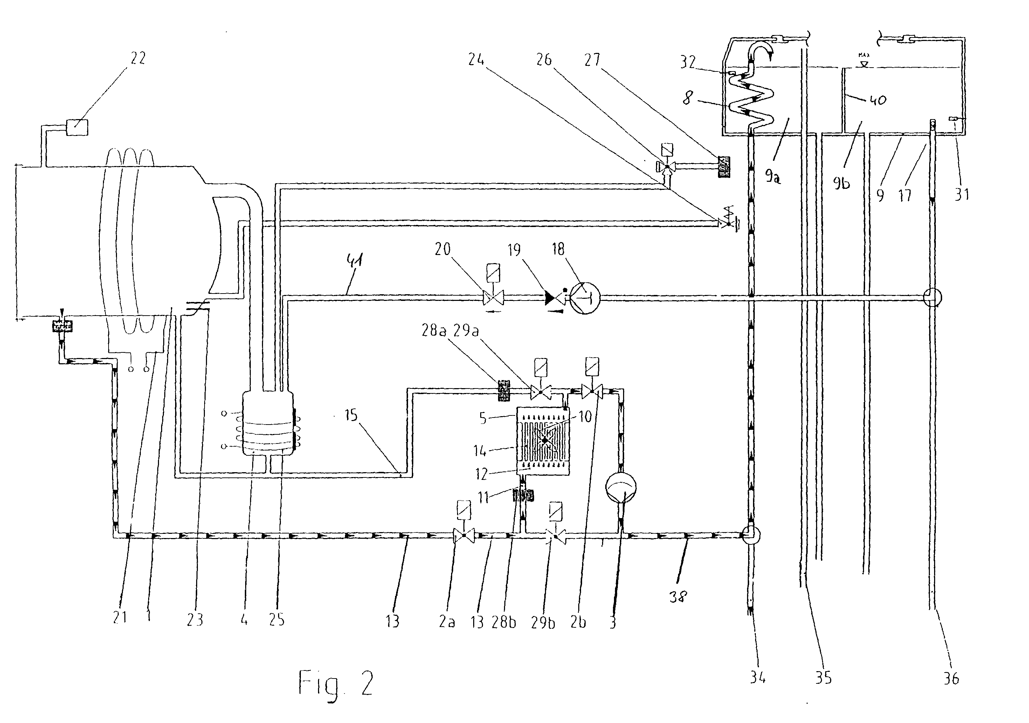

Ein möglicher Weg führt den Dampf über eine zweite Dampfleitung 13, ein drittes Magnetventil 2a, einen zweiten Druckablaßfilter 28b, den Kondensator 5 und ein zweites Magnetventil 2b zur Vakuumpumpe 3. Diese Evakuierungsbetriebsweise wird eingehend in Fig. 2 dargestellt.

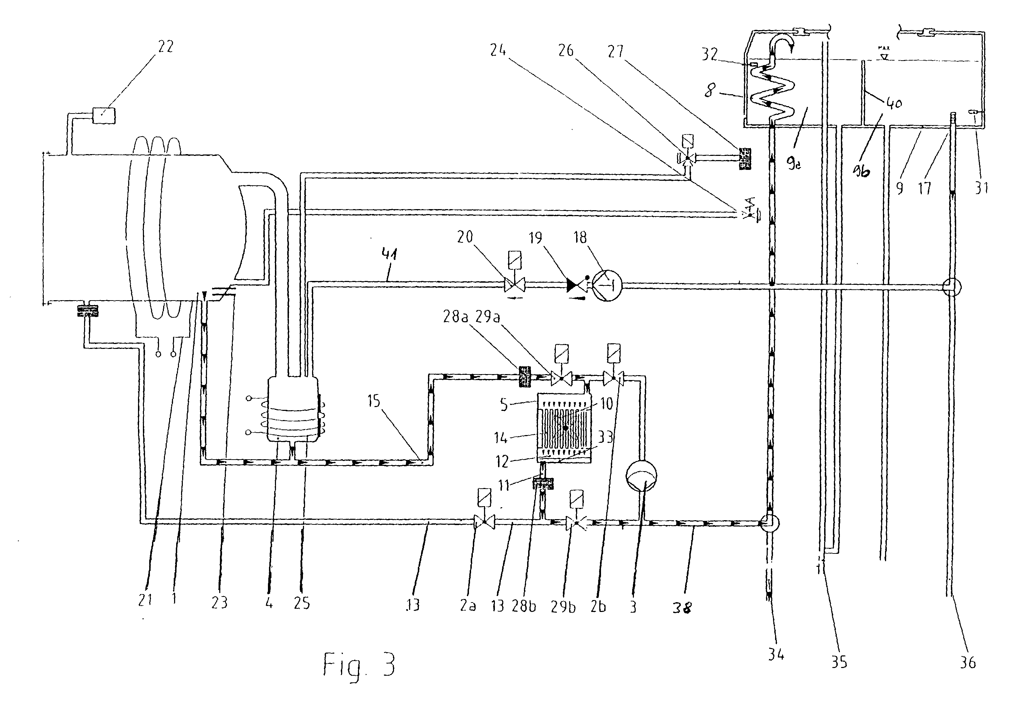

Ein zweiter möglicher Weg des Dampfs aus dem Druckkessel 1 führt über die erste Dampfleitung 15, über einen ersten Druckablaßfilter 28a, ein erstes Magnetventil 29a, den Kondensator 5, ein Kondensatsammelgefäß 12, den zweiten Druckablaßfilter 28b und das vierte Magnetventil 29b zu einem Wasserbehälter 9. Diese Kondensat-Druckentleerung wird eingehender in Fig. 3 dargestellt.

Zwischen dem ersten Magnetventil 29a und dem zweiten Magnetventil 2b ist eine Rohrabzweigung zu dem Kondensator 5 (hier mit Kondensatsammelbehälter 12) vorgesehen.

Beide möglichen Wege sind in Fig. 1 durch kleine Pfeile in den Rohrleitungen gekennzeichnet.

Der Kondensator 5 weist einen Luftkühler 14 mit einem Ventilator 10 sowie ein Kondensatsammelgefäß 12 auf. Grundsätzlich ist es aber auch denkbar, daß sich das Kondensat im Kondensator 5 sammelt und kein spezieller Auffangraum für das Kondensat vorgesehen ist.

Die zweite Dampfleitung 13 ist unten an das Kondensatsammelgefäß 12 angeschlossen und bildet in einem kleinen Abschnitt gleichzeitig einen Kondensatablauf 11.

Ein Wasserbehälter 9 weist eine Abwasserseite 9a und eine Frischwasserseite 9b auf. Die Frischwasserseite 9b ist über eine Wasserzuleitung 36 mit einem Wasserzulauf aus einer hier nicht dargestellten Wasseraufbereitungsanlage verbunden, wobei ein Wasserzulauffilter 17 zwischengeschaltet ist. Die Wasseraufbereitungsanlage stellt demineralisierts oder destilliertes Wasser zur Verfügung. Dabei ist es möglich, daß die Frischwasserversorgung alternativ aus dem Wasserbehälter 9 oder durch den direkten Anschluß oder durch einen Mischbetrieb erfolgen kann. Auch ist eine Handbefüllung möglich.

Der Wasserstand der Frischwasserseite 9b des Wasserbehälters 9 wird mittels eines ersten Schwimmerschalter 31 geschaltet. Der Wasserstand der Abwasserseite 9a wird mittels eines zweiten Schimmerschalters 32 gesteuert. Zur Sicherheit ist zusätzlich ein Überlaufrohr 35 vorgesehen.

Die Abwasserseite 9a und die Frischwasserseite 9b sind über ein Überlaufwehr 40 miteinander verbunden. Der Frischwasserseite 9b kann über eine Speisepumpe 18 Wasser für den Dampferzeuger 4 entnommen werden. Alternativ oder gleichzeitig kann die Speisepumpe 18 auch Wasser über die Wasserzuleitung 36 von einer externen Quelle (z.B. Wasseraufbereitungsanlage oder externer Vorratsbehälter) ansaugen.

Eine Wasserleitung 41 weist hinter der Speisepumpe 18 ein Rückschlagventil 19 und als Wasserleitungsventil 20 ein Magnetventil auf.

In einer alternativen Ausgestaltung erfolgt die Frischwasserzufuhr mit Hilfe eines Überdruckes; auf die Speisepumpe kann verzichtet werden, oder sie kann entsprechend kleiner ausgelegt werden. So kann durch eine höhere Anordnung des Wasserbehälters 9 ein statischer Druck erzeugt werden, der das Frischwasser fördert. Auch liegen hinter Wasseraufbereitungsanlagen regelmäßig etwas höhere Drücke an, so daß dieser Druck zur Frischwasserförderung verwendbar ist.

Die Kondensatleitung 38 verläuft durch die Abwasserseite 9a des Wasserbehälters 9 und ist dort als Kondensatorschlange 8 ausgebildet.

Fig. 1 zeigt die grundsätzlichen Wege, die der Dampf in der erfindungsgemäßen Vorrichtung nehmen kann.

Je nach Stellung der Magnetventile 2a, 2b, 29a, 29b wird einmal mittels des dritten Magnetventils 2a Dampf direkt in den Kondensator 5 geleitet. Der Dampf wird im Kondensator 5 kondensiert. Die Evakuierung des Systems erfolgt über das zweite Magnetventils 2b mittels der Vakuumpumpe 3 (siehe Fig. 2).

Oder es wird über das erste Magnetventils 29a Dampf im Kondensator 5 zur Kondensation gebracht. Infolge Überdrucks im Druckkessel wird das Kondensat über das vierte Magnetventil 29b entleert. Dies kann wahlweise in den Abwasserteil 9a des Wasserbehälters 9 oder auch durch den Kondensatablaß 34 direkt den Abfluß erfolgen. Grundsätzlich ist es auch möglich, einen Mischbetrieb der beiden Möglichkeiten des Abwasserablasses zu verwenden.

Anhand der Fig. 2 soll der Betrieb der erfindungsgemäßen Vorrichtung erläutert werden. Die Pfeile in den Leitungen zeigen den Weg des Dampfes bzw. der Luft beim Evakuieren des Druckkessels 1 an.

Bei Programmstart wird der Druckkessel 1 durch Öffnen des dritten Magnetventils 2a und des zweiten Magnetventils 2b und Einschalten der Vakuumpumpe 3 auf einen vorbestimmten Druck evakuiert. Anschließend wird das dritte Magnetventil 2a und das zweite Magnetventil 2b geschlossen und aus dem Dampferzeuger 4 Dampf in den Druckkessel 1 eingeleitet, bis ein vorgegebener Druck erreicht ist. Durch erneutes Öffnen des dritten Magnetventils 2a und des zweiten Magnetventils 2b und Einschalten der Vakuumpumpe 3 wird das Dampf/Luftgemisch wiederum bis zu einem definierten Druck abgesaugt. Dabei wird der Wasserdampf im Kondensator 5 kondensiert und im Kondensatsammelgefäß 12 gesammelt, so daß die Vakuumpumpe 3 nur Luft fördert. Die Kühlung des Kondensators 5 erfolgt durch Luft, wobei die Kühlwirkung durch Kühlrippen am Kondensator 5 und durch den Ventilator 10 verstärkt wird.

In Abhängigkeit von der Beladungsart des Druckkessels 1 kann der genannte Vorgang beliebig oft wiederholt werden, bis der geforderte Verdünnungsgrad der Luft innerhalb des Druckkessels 5 erreicht ist. Der notwendige Druckablaß mit der erfindungsgemäßen Kondensatentleerung wird in Fig. 3 beschrieben.

An den eigentlichen Sterilisationsprozeß schließt sich eine Vakuum-Trocknungsphase an. Dabei wird der Druckkessel 1 wieder evakuiert und der Dampf im Kondensator 5 gekühlt und gesammelt. Am Ende der Trocknungsphase wird dieses Kondensat ebenfalls, wie weiter unten beschrieben werden wird, abgeleitet.

Durch die Kondensation des abströmenden Dampfes im Kondesator 5 wird eine zusätzliche Pumpwirkung hervorgerufen. Dadurch werden die Evakuierungszeiten verkürzt, Trocknungsergebnisse verbessert bzw. kann bei hohem Verdünnungsgrad der Luft im Druckkessel 1 die Vakuumpumpe 3 nach dem Initialisieren der Dampfströmung teilweise oder ganz abgeschaltet werden.

Die Kondensation wird im vorliegenden Ausführungsbeispiel durch die Kondensatorschlange 8 unterstützt, die hinter dem Kondensator 5 angeordnet ist. Die Kondensatorschlange 8 wird durch das Wasser im Wasserbehälter 9 gekühlt. Diese zusätzliche Kühlung verringert die Temperatur des austretenden Kondensates.

Nach einer gewissen Zeit muß das angefallene Kondensat aus dem Kondensatsammelgefäß 12 entfernt werden. Wie oben beschrieben, ist in Fig. 3 dargestellt, daß nach dem Evakuieren das dritte Magnetventil 2a und das zweite Magnetventil 2b geschlossen werden. Zur Kondensatentleerung wird ein erstes Magnetventil 29a und ein viertes Magnetventil 29b in einer Kondensatleitung 38 geöffnet. Nunmehr besteht eine Verbindung vom Druckkessel 1 zur Kondensatleitung 38. Die Pfeile in den Rohren geben dabei die Strömungsrichtung an.

Der im Druckkessel 1 herrschende Überdruck beaufschlagt über die erste Dampfleitung 15 den Kondensator 5 und damit das Kondensammelgefäß 12. Der Überdruck ist groß genug, um einen statischen Druck in der Kondensatleitung 38 zu überwinden und das Kondensat in die Abwasserseite 9a des Wasserbehälters 9 zu drücken. Die erste Dampfleitung 15 und die entsprechenden Magnetventile am Kondensator 5 bilden zusammen ein Mittel, um das Kondensat durch Überdruck aus dem Kondensatsammelgefäß 12 zu entleeren.

Grundsätzlich sind in alternativen Ausführungen aber auch andere Leitungskonfigurationen und / oder Druckquellen (z.B. externe Druckleitung, Druck aus einem anderem Gerät) möglich, um eine Druckentleerung des Kondensators 5 und /oder des Kondensatzsammelgefäßes 12 zu erreichen.

Wenn es die Betriebsbedingungen erfordern, kann das Kondensat anstatt in den Wasserbehälter 9, über eine Kondensatablaufleitung 34 abgelassen werden.

Die erfindungsgemäße Vorrichtung und das erfindungsgemäße Verfahren zur Druckentleerung des Kondensates wurde hier abhand Dampfsterilisators beschrieben, der nach einem fraktionierten Vakuumverfahren arbeitet. Grundsätzlich ist die Lehre der Erfindung aber auch auf andere Dampfsterilisatoren und deren Betriebsweisen anwendbar.

## Patentansprüche

1. Dampfsterilisator mit einem Druckkessel und einem Kondensator für aus dem Druckkessel stammenden Dampf,
**gekennzeichnet durch**
ein Mittel zur Entleerung von Kondensat **durch** Überdruck aus dem Kondensator (5) und / oder mindestens einem Kondensatsammelgefäß (12).

2. Dampfsterilisator nach Anspruch 1, **dadurch gekennzeichnet, daß** der Kondensator (5) und / oder das Kondensatsammelgefäß (12) über eine erste Dampfleitung (15) vom Druckkessel (1) mit Überdruck beaufschlagbar ist.

3. Dampfsterilisator nach Anspruch 1 oder 2, **dadurch gekennzeichnet, daß** der Kondensator (5) und / oder das Kondensatorsammelgefäß (12) über eine externe Druckquelle mit Überdruck beaufschlagbar ist.

4. Dampfsterilisator nach mindestens einem der vorhergehenden Ansprüche, **dadurch gekennzeichnet, daß** das Kondensat durch Überdruck in einen Wasserbehälter (9) und / oder eine Kondensatablaufleitung (34) förderbar ist.

5. Dampfsterilisator nach Anspruch 4, **dadurch gekennzeichnet, daß** der Wasserbehälter (9) einen Abwasserteil (9a) und einen Frischwasserteil (9b) aufweist.

6. Dampfsterilisator nach Anspruch 5, **dadurch gekennzeichnet, daß** das Kondensat in den, insbesondere auf höherliegenden, Abwasserteil (9a) des Wasserbehälters (9) förderbar ist.

7. Dampfsterilisator nach mindestens einem der Ansprüche 4 bis 6, **dadurch gekennzeichnet, daß** der Abwasserteil (9a) und der Frischwasserteil (9b) über ein Überlaufwehr (40) miteinander in Verbindung stehen.

8. Dampfsterilisator nach mindestens einem der vorhergehenden Ansprüche, **dadurch gekennzeichnet, daß** der Kondensator (5) Kühlflächen aufweist, denen mindestens ein Ventilator (10) und/oder eine Wärmepumpe und/oder Peltierelemente zugeordnet sind.

9. Dampfsterilisator nach mindestens einem der vorangehenden Ansprüche, **dadurch gekennzeichnet, daß** über die zweite Dampfleitung (13) eine Vakuumpumpe (3) mit dem Druckkessel (1) verbindbar ist.

10. Dampfsterilisator nach Anspruch 9, **dadurch gekennzeichnet, daß** die Vakuumpumpe (3) als Membranpumpe ausgebildet ist.

11. Dampfsterilisator nach mindestens einem der vorhergehenden Ansprüche, **dadurch gekennzeichnet, daß** eine vom Druckkessel (1) abgehende zweite Dampfleitung (13) unterhalb des Luftkühlers (14) am Kondensatsammelgefäß (12) angeschlossen ist und daß ein Kondensatablauf unterhalb des Luftkühlers (14) und oberhalb des Kondensatsammelgefäßes (12) vorgesehen ist.

12. Dampfsterilisator nach mindestens einem der vorhergehenden Ansprüche, **dadurch gekennzeichnet, daß** eine vom Druckkessel (1) abgehende zweite Dampfleitung (13) und ein Kondensatablauf unterhalb des Kondensators (5) am Kondensatsammelgefäß (12) vorgesehen sind.

13. Dampfsterilisator nach mindestens einem der vorhergehenden Ansprüche, **dadurch gekennzeichnet, daß** eine Kondensatleitung (38) hinter dem Kondensator (5) oder dem Kondensatsammelgefäß (12) durch den Wasserbehälter (9) verläuft und in diesem Bereich als Kondensatorschlange (8) ausgebildet ist.

14. Dampfsterilisator nach mindestens einem der vorhergehenden Ansprüche, **dadurch gekennzeichnet, daß** Wasser durch eine Speisepumpe (18) aus dem Wasserbehälter (9) und / oder aus einer Wasserzuleitung (36) zum Druckkessel (1) pumpbar ist.

15. Dampfsterilisator nach mindestens einem der vorhergehenden Ansprüche, **dadurch gekennzeichnet, daß** Wasser aus dem Wasserbehälter (9) und / oder aus einer Wasserzuleitung aufgrund einer Druckdifferenz zum Druckkessel (1) förderbar ist.

16. Dampfsterilisator nach Anspruch 15, **dadurch gekennzeichnet, daß** die Druckdifferenz durch ein Vakuum im Druckkessel (1) erzeugbar ist.

17. Verfahren zur Dampfsterilisation in einem Druckkessel,
**dadurch gekennzeichnet, daß**
Dampf in einem Kondensator (5) kondensiert wird und das Kondensat aus dem Kondensator (5) und / oder mindestens einem Kondensatsammelgefäß (12) durch Überdruck entleert wird.

18. Verfahren nach Anspruch 17, **dadurch gekennzeichnet, daß** der Überdruck zur Kondensatentleerung aus dem Druckkessel (5) stammt.

19. Verfahren nach Anspruch 17 oder 18, **dadurch gekennzeichnet, daß** der Überdruck zur Kondensatentleerung aus einer externen Druckquelle stammt.

## Claims

1. Steam sterilizer having a pressure chamber and a condenser for steam originating from the pressure chamber, **characterized by** a means for emptying condensate from the condenser (5) and/or at least one condensate collecting vessel (12) by positive pressure.

2. Steam sterilizer according to Claim 1, **characterized in that** positive pressure can be applied to the condenser (5) and/or the condensate collecting vessel (12) via a first steam line (15) from the pressure chamber (1).

3. Steam sterilizer according to Claim 1 or 2, **characterized in that** positive pressure can be applied to the condenser (5) and/or the condenser collecting vessel (12) via an external pressure source.

4. Steam sterilizer according to at least one of the preceding claims, **characterized in that** the condensate can be delivered into a water tank (9) and/or a condensate discharge line (34) by positive pressure.

5. Steam sterilizer according to Claim 4, **characterized in that** the water tank (9) has a wastewater part (9a) and a freshwater part (9b).

6. Steam sterilizer according to Claim 5, **characterized in that** the condensate can be delivered into the wastewater part (9a), which in particular is at a higher level, of the water tank (9).

7. Steam sterilizer according to at least one of Claims 4 to 6, **characterized in that** the wastewater part (9a) and the freshwater part (9b) are connected to one another via an overflow weir (40).

8. Steam sterilizer according to at least one of the preceding claims, **characterized in that** the condenser (5) has cooling surfaces, to which at least one fan (10) and/or a heat pump and/or Peltier elements are assigned.

9. Steam sterilizer according to at least one of the preceding claims, **characterized in that** a vacuum pump (3) can be connected to the pressure chamber (1) via the second steam line (13).

10. Steam sterilizer according to Claim 9, **characterized in that** the vacuum pump (13) is designed as a diaphragm pump.

11. Steam sterilizer according to at least one of the preceding claims, **characterized in that** a second steam line (13) branching off from the pressure chamber (1) is connected below the air cooler (14) to the condensate collecting vessel (12), and **in that** a condensate discharge is provided below the air cooler (14) and above the condensate collecting vessel (12).

12. Steam sterilizer according to at least one of the preceding claims, **characterized in that** a second steam line (13) branching off from the pressure vessel (1) and a condensate discharge are provided below the condenser (5) on the condensate collecting vessel (12).

13. Steam sterilizer according to at least one of the preceding claims, **characterized in that** a condensate line (38) runs downstream of the condenser (5) or the condensate collecting vessel (12) through the water tank (9) and is designed as a condenser coil (8) in this region.

14. Steam sterilizer according to at least one of the preceding claims, **characterized in that** water can be pumped from the water tank (9) and/or from a water feed line (36) to the pressure chamber (1) by a feed pump (18).

15. Steam sterilizer according to at least one of the preceding claims, **characterized in that** water can be delivered from the water tank (9) and/or from a water feed line to the pressure chamber (1) on account of a pressure difference.

16. Steam sterilizer according to Claim 15, **characterized in that** the pressure difference can be produced by a vacuum in the pressure chamber (1).

17. Method of sterilizing steam in a pressure chamber, **characterized in that** steam is condensed in a condenser (5) and the condensate is emptied from the condenser (5) and/or at least one condensate collecting vessel (12) by positive pressure.

18. Method according to Claim 17, **characterized in that** the positive pressure for the condensate emptying originates from the pressure chamber (1).

19. Method according to Claim 17 or 18, **characterized in that** the positive pressure for the condensate emptying originates from an external pressure source.

## Revendications

1. Stérilisateur à vapeur avec un réservoir sous pression et un condensateur pour la vapeur provenant du réservoir sous pression, **caractérisé par** un moyen destiné à vider du condenseur (5) le condensat par surpression et/ou au moins un récipient collecteur de condensat (12).

2. Stérilisateur à vapeur selon la revendication 1 **caractérisé en ce que** le condensateur (5) et/ou le récipient collecteur de condensat (12) peut être alimenté en surpression par une première conduite de vapeur (15) provenant du réservoir sous pression (1).

3. Stérilisateur à vapeur selon la revendication 1 ou 2 **caractérisé en ce que** le condensateur (5) et/ou le récipient collecteur de condensat (12) peut être alimenté en surpression par une source de pression externe.

4. Stérilisateur à vapeur selon au moins l'une des revendications précédentes **caractérisé en ce que** le condensat peut être transporté par surpression dans un réservoir d'eau (9) et/ou par une conduite de déversement du condensat (34).

5. Stérilisateur à vapeur selon la revendication 4 **caractérisé en ce que** le réservoir d'eau (9) présente une partie pour l'eau usagée (9a) et une partie pour l'eau fraîche (9b).

6. Stérilisateur à vapeur selon la revendication 5 **caractérisé en ce que** le condensat peut être transporté dans la partie pour l'eau usagée (9a) du réservoir d'eau (9), en particulier placée en hauteur.

7. Stérilisateur à vapeur selon au moins l'une des revendications précédentes 4 à 6 **caractérisé en ce que** la partie pour l'eau usagée (9a) et la partie pour l'eau fraîche (9b) sont en liaison l'une avec l'autre par un déversoir de superficie (40).

8. Stérilisateur à vapeur selon au moins l'une des revendications précédentes **caractérisé en ce que** le condensateur (5) présente des surfaces de refroidissement auxquelles sont associées au moins un ventilateur (10) et/ou une pompe à chaleur et/ou des éléments Peltier.

9. Stérilisateur à vapeur selon au moins l'une des revendications précédentes **caractérisé en ce qu'**une pompe à vide (3) peut être reliée au réservoir sous pression (1) par la deuxième conduite de vapeur (13).

10. Stérilisateur à vapeur selon la revendication 9 **caractérisé en ce que** la pompe à vide (3) est constituée comme une pompe à diaphragme.

11. Stérilisateur à vapeur selon au moins l'une des revendications précédentes **caractérisé en ce qu'**une deuxième conduite de vapeur (13) sortant du réservoir sous pression (1) est raccordée au récipient collecteur de condensat (12) au-dessous du refroidisseur à air (14) et qu'un déversoir du condensat est prévu au-dessous du refroidisseur à air (14) et au-dessus du récipient collecteur de condensat (12).

12. Stérilisateur à vapeur selon au moins l'une des revendications précédentes **caractérisé en ce qu'**une deuxième conduite de vapeur (13) sortant du réservoir sous pression (1) et un déversoir du condensat au-dessous du condensateur (5) sont prévus sur le récipient collecteur de condensat (12).

13. Stérilisateur à vapeur selon au moins l'une des revendications précédentes **caractérisé en ce qu'**une conduite de condensat (38) traverse le réservoir d'eau (9) derrière le condensateur (5) ou derrière le récipient collecteur de condensat (12) et est constituée dans cette zone comme un serpentin de condensateur (8).

14. Stérilisateur à vapeur selon au moins l'une des revendications précédentes **caractérisé en ce que** de l'eau peut être pompée du réservoir d'eau (9) et/ou d'une conduite d'amenée d'eau (36) vers le réservoir sous pression (1) par une pompe d'alimentation (18).

15. Stérilisateur à vapeur selon au moins l'une des revendications précédentes **caractérisé en ce que**, du fait d'une différence de pression, de l'eau peut être transportée du réservoir d'eau (9) et/ou d'une conduite d'amenée d'eau vers le réservoir sous pression (1).

16. Stérilisateur à vapeur selon la revendication 15 **caractérisé en ce que** la différence de pression peut être produite par un vide dans le réservoir sous pression (1).

17. Procédé de stérilisation à la vapeur dans un réservoir sous pression **caractérisé en ce que** de la vapeur est condensée dans un condensateur (5) et le condensat est vidé par surpression du condensateur (5) et/ou d'au moins un récipient collecteur de condensat (12).

18. Procédé selon la revendication 17 **caractérisé en ce que** la surpression servant à vider le condensat provient du réservoir sous pression (5).

19. Procédé selon la revendication 17 ou 18 **caractérisé en ce que** la surpression servant à vider le condensat provient d'une source de pression externe.
